# EUROPEAN PATENT APPLICATION

(11) **EP 4 717 185 A1**
(43) Date of publication of application: **01.04.2026**
(21) Application number: 25197872.2
(22) Date of filing: 25.08.2025
(51) Int. Cl.: A61B 8/08, A61B 8/00, A61B 8/06

(54) **ULTRASOUND DIAGNOSTIC APPARATUS AND METHOD OF CONTROLLING ULTRASOUND DIAGNOSTIC APPARATUS**

(30) Priority: 26.09.2024 JP 2024167576
(71) Applicant: FUJIFILM Corporation, Tokyo 106-8620 (JP)
(72) Inventor: TAKASHIMA, Kaito, Kaisei (JP)
(74) Representative: Meissner Bolte Partnerschaft mbB

(57) **Abstract**

Provided are an ultrasound diagnostic apparatus and a method of controlling an ultrasound diagnostic apparatus that can accurately measure a cardiac function using ultrasound images representing a plurality of types of cross sections.

An ultrasound diagnostic apparatus includes: an image acquisition unit that acquires first and second ultrasound images in which different cross sections of a heart are imaged, by transmitting and receiving ultrasound beams using an ultrasound probe; a feature extraction unit that extracts a feature of an anatomical structure from each of the first ultrasound image and the second ultrasound image; a consistency calculation unit that calculates consistency between the features of the anatomical structures in the first ultrasound image and the second ultrasound image extracted by the feature extraction unit; an appropriateness calculation unit that calculates appropriateness calculated for each of the first ultrasound image and the second ultrasound image; a measurement instruction unit that starts measurement in a case in which the consistency is equal to or greater than a predetermined consistency threshold value, and both the appropriateness of the first ultrasound image and the appropriateness of the second ultrasound image are equal to or greater than a predetermined appropriateness threshold value.

## Description

### BACKGROUND OF THE INVENTION

### 1. Field of the Invention

The present invention relates to an ultrasound diagnostic apparatus that captures an ultrasound image of a heart of a subject and a method of controlling the ultrasound diagnostic apparatus.

### 2. Description of the Related Art

In the related art, an ultrasound image representing a tomographic plane of a heart of a subject is captured using a so-called ultrasound diagnostic apparatus, and the ultrasound image is used to measure cardiac functions such as a so-called left ventricular ejection fraction (LVEF), a left ventricular end-diastolic volume, a left ventricular end-systolic volume, a left ventricular local wall motion, a global longitudinal strain (GLS), a mitral annular plane systolic excursion (MAPSE), a tricuspid annular plane systolic excursion (TAPSE), a cardiac output, and a single stroke volume.

A user usually checks the captured ultrasound image to determine whether or not a cross section suitable for measuring a target cardiac function is captured, but it may be difficult to make this determination depending on a skill level of the user. Therefore, for example, as disclosed in JP2017-164077A, a technology for automatically determining a type of the cross section represented by the captured ultrasound image has been developed.

### SUMMARY OF THE INVENTION

In a case of measuring the left ventricular ejection fraction as the cardiac function, the measurement is often performed using two types of ultrasound images, that is, an ultrasound image representing a so-called apical four-chamber cross section and an ultrasound image representing an apical two-chamber cross section. In a case in which the cardiac function is measured using a plurality of ultrasound images representing a plurality of types of cross sections as described above, even in a case in which the type of the cross section represented by each ultrasound image is automatically determined by the technology of JP2017-164077A, for example, in a case in which a position or an inclined angle of the ultrasound probe deviates in a case in which ultrasound images representing different types of cross sections are captured, the cardiac function may not be accurately measured.

The present invention has been made in order to solve such a problem in the related art, and an object of the present invention is to provide an ultrasound diagnostic apparatus and a method of controlling an ultrasound diagnostic apparatus that can accurately measure a cardiac function using ultrasound images representing a plurality of types of cross sections.

The object described above can be achieved with the following configurations.
[1] An ultrasound diagnostic apparatus comprising: an ultrasound probe; a monitor; an image acquisition unit that acquires first and second ultrasound images in which different cross sections of a heart of a subject are imaged, by transmitting and receiving ultrasound beams using the ultrasound probe; a feature extraction unit that extracts a feature of an anatomical structure of the heart from each of the first ultrasound image and the second ultrasound image that are acquired by the image acquisition unit; a consistency calculation unit that calculates consistency between the feature of the anatomical structure in the first ultrasound image extracted by the feature extraction unit and the feature of the anatomical structure in the second ultrasound image extracted by the feature extraction unit; an appropriateness calculation unit that calculates appropriateness as a target for measurement for each of the first ultrasound image and the second ultrasound image; a measurement unit that measures a cardiac function of the subject based on the first ultrasound image and the second ultrasound image; and a measurement instruction unit that instructs the measurement unit to execute measurement in a case in which the consistency calculated by the consistency calculation unit between the first ultrasound image and the second ultrasound image is equal to or greater than a predetermined consistency threshold value, and both the appropriateness calculated by the appropriateness calculation unit for the first ultrasound image and the appropriateness calculated by the appropriateness calculation unit for the second ultrasound image are equal to or greater than a predetermined appropriateness threshold value.
[2] The ultrasound diagnostic apparatus according to [1], in which the first ultrasound image and the second ultrasound image are images in which two of an apical two-chamber cross section, an apical three-chamber cross section, an apical four-chamber cross section, or an apical five-chamber cross section are imaged.
[3] The ultrasound diagnostic apparatus according to [1] or [2], in which the measurement unit measures a left ventricular ejection fraction or a cardiac output as the cardiac function of the subject.
[4] The ultrasound diagnostic apparatus according to any one of [1] to [3], in which the image acquisition unit acquires a third ultrasound image in which a cross section of the heart different from both the cross sections in the first ultrasound image and the second ultrasound image is imaged, the feature extraction unit extracts a feature of an anatomical structure of the heart from the third ultrasound image, the consistency calculation unit calculates consistency between the feature of the anatomical structure in the first ultrasound image and the feature of the anatomical structure in the second ultrasound image and the feature of the anatomical structure in the third ultrasound image, the appropriateness calculation unit calculates appropriateness as a target for measurement for the third ultrasound image, and the measurement instruction unit starts further measurement of the cardiac function by the measurement unit in a case in which the consistency between the feature of the anatomical structure in the first ultrasound image and the feature of the anatomical structure in the second ultrasound image and the feature of the anatomical structure in the third ultrasound image is equal to or greater than the predetermined consistency threshold value, and the appropriateness calculated by the appropriateness calculation unit for the third ultrasound image is equal to or greater than the predetermined appropriateness threshold value.
[5] The ultrasound diagnostic apparatus according to any one of [1] to [4], in which the feature extraction unit extracts a length of a long axis of a left ventricle as the feature of the anatomical structure of the heart.
[6] The ultrasound diagnostic apparatus according to [5], in which the consistency threshold value is 90%.
[7] The ultrasound diagnostic apparatus according to any one of [1] to [4], in which the feature extraction unit extracts a position of a cardiac apex as the feature of the anatomical structure of the heart.
[8] The ultrasound diagnostic apparatus according to [7], in which the consistency threshold value is 90%.
[9] The ultrasound diagnostic apparatus according to any one of [1] to [8], further comprising: an adjustment instruction unit that instructs a user to adjust at least one of a scanning position of the ultrasound probe, a posture of the subject, or a breathing method of the subject in a case in which, for a predetermined time, the consistency calculated by the consistency calculation unit between the first ultrasound image and the second ultrasound image is less than the predetermined consistency threshold value or any of the appropriateness calculated by the appropriateness calculation unit for the first ultrasound image or the second ultrasound image is less than the predetermined appropriateness threshold value.
[10] A method of controlling an ultrasound diagnostic apparatus, the method comprising: acquiring first and second ultrasound images in which different cross sections of a heart of a subject are imaged, by transmitting and receiving ultrasound beams using an ultrasound probe; extracting a feature of an anatomical structure of the heart from each of the first ultrasound image and the second ultrasound image; calculating consistency between the extracted feature of the anatomical structure in the first ultrasound image and the extracted feature of the anatomical structure in the second ultrasound image; calculating appropriateness as a target for measurement for each of the first ultrasound image and the second ultrasound image; and executing measurement of a cardiac function of the subject in a case in which the consistency calculated between the first ultrasound image and the second ultrasound image is equal to or greater than a predetermined consistency threshold value, and both the appropriateness calculated for the first ultrasound image and the appropriateness calculated for the second ultrasound image are equal to or greater than a predetermined appropriateness threshold value.

The aspect of the present invention provides the ultrasound diagnostic apparatus comprising: the ultrasound probe; the monitor; the image acquisition unit that acquires the first and second ultrasound images in which different cross sections of the heart of the subject are imaged, by transmitting and receiving the ultrasound beams using the ultrasound probe; the feature extraction unit that extracts the feature of the anatomical structure of the heart from each of the first ultrasound image and the second ultrasound image that are acquired by the image acquisition unit; the consistency calculation unit that calculates consistency between the feature of the anatomical structure in the first ultrasound image extracted by the feature extraction unit and the feature of the anatomical structure in the second ultrasound image extracted by the feature extraction unit; the appropriateness calculation unit that calculates the appropriateness as the target for measurement for each of the first ultrasound image and the second ultrasound image; the measurement unit that measures the cardiac function of the subject based on the first ultrasound image and the second ultrasound image; and the measurement instruction unit that instructs the measurement unit to execute the measurement in a case in which the consistency calculated by the consistency calculation unit between the first ultrasound image and the second ultrasound image is equal to or greater than the predetermined consistency threshold value, and both the appropriateness calculated by the appropriateness calculation unit for the first ultrasound image and the appropriateness calculated by the appropriateness calculation unit for the second ultrasound image are equal to or greater than the predetermined appropriateness threshold value, so that it is possible to accurately measure the cardiac function using the ultrasound images representing the plurality of types of cross sections.

### BRIEF DESCRIPTION OF THE DRAWINGS

FIG. 1 is a block diagram showing a configuration of an ultrasound diagnostic apparatus according to Embodiment 1 of the present invention.
FIG. 2 is a block diagram showing an internal configuration of a transmission/reception circuit in Embodiment 1 of the present invention.
FIG. 3 is a block diagram showing an internal configuration of an image generation unit in Embodiment 1 of the present invention.
FIG. 4 is a schematic diagram showing an example of a first ultrasound image representing an apical four-chamber cross section.
FIG. 5 is a schematic diagram showing an example of a second ultrasound image representing an apical two-chamber cross section.
FIG. 6 is a diagram showing a display example of appropriateness calculated for the first ultrasound image.
FIG. 7 is a diagram showing a display example of appropriateness and consistency calculated for the second ultrasound image.
FIG. 8 is a flowchart showing an operation of the ultrasound diagnostic apparatus according to Embodiment 1 of the present invention.
FIG. 9 is a flowchart showing an operation of acquiring the first ultrasound image that is a target for measurement in Embodiment 1 of the present invention.
FIG. 10 is a flowchart showing an operation of acquiring the second ultrasound image that is a target for measurement in Embodiment 1 of the present invention.
FIG. 11 is a block diagram showing a configuration of an ultrasound diagnostic apparatus according to Embodiment 2 of the present invention.

### DESCRIPTION OF THE PREFERRED EMBODIMENTS

Hereinafter, embodiments of the present invention will be described with reference to the accompanying drawings.

The following configuration requirements are described based on a representative embodiment of the present invention, but the present invention is not limited to the embodiment.

In the present specification, a numerical range represented by "to" means a range including numerical values described before and after "to", both ends inclusive, as a lower limit value and an upper limit value.

In the present specification, "the same" includes an error range generally allowed in the technical field.

### Embodiment 1

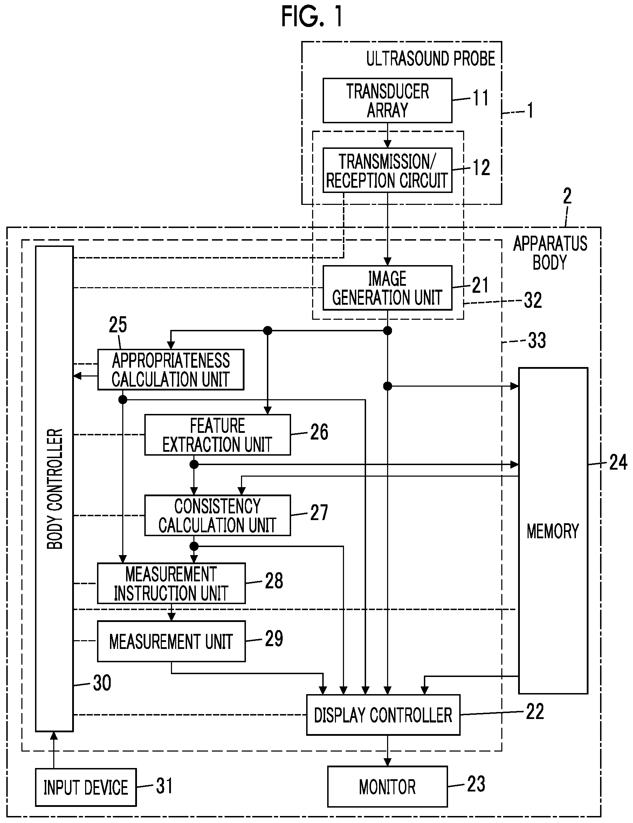

FIG. 1 shows a configuration of an ultrasound diagnostic apparatus according to the embodiment of the present invention.

The ultrasound diagnostic apparatus comprises an ultrasound probe 1 and an apparatus body 2 that are connected to each other by so-called wired communication or so-called wireless communication.

The ultrasound probe 1 comprises a transducer array 11 and a transmission/reception circuit 12 connected to the transducer array 11.

The apparatus body 2 comprises an image generation unit 21 connected to the transmission/reception circuit 12. In the apparatus body 2, a display controller 22 and a monitor 23 are sequentially connected to the image generation unit 21. A memory 24 and an appropriateness calculation unit 25 are connected to the image generation unit 21. Further, a feature extraction unit 26 is connected to the image generation unit 21. The feature extraction unit 26 is connected to the memory 24 and a consistency calculation unit 27. The memory 24 is connected to the consistency calculation unit 27. Further, a measurement instruction unit 28 is connected to the appropriateness calculation unit 25 and the consistency calculation unit 27. A measurement unit 29 is connected to the measurement instruction unit 28. In addition, the memory 24, the appropriateness calculation unit 25, the consistency calculation unit 27, and the measurement unit 29 are connected to the display controller 22. In addition, a body controller 30 is connected to the transmission/reception circuit 12, the image generation unit 21, the display controller 22, the memory 24, the appropriateness calculation unit 25, the feature extraction unit 26, the consistency calculation unit 27, the measurement instruction unit 28, and the measurement unit 29. An input device 31 is connected to the body controller 30.

The transmission/reception circuit 12 and the image generation unit 21 constitute an image acquisition unit 32. In addition, the image generation unit 21, the display controller 22, the appropriateness calculation unit 25, the feature extraction unit 26, the consistency calculation unit 27, the measurement instruction unit 28, the measurement unit 29, and the body controller 30 constitute a processor 33 for the apparatus body 2.

The transducer array 11 of the ultrasound probe 1 includes a plurality of ultrasound transducers that are one-dimensionally or two-dimensionally arranged. In accordance with a drive signal supplied from the transmission/reception circuit 12, each of the ultrasound transducers transmits ultrasound and receives an ultrasound echo from a subject to output a signal based on the ultrasound echo. Each ultrasound transducer is configured by, for example, forming electrodes at both ends of a piezoelectric material consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), and the like.

The image acquisition unit 32, which is composed of the transmission/reception circuit 12 and the image generation unit 21, acquires ultrasound images of a plurality of frames as a moving image in which a heart of the subject is imaged, by transmitting and receiving ultrasound beams using the ultrasound probe 1.

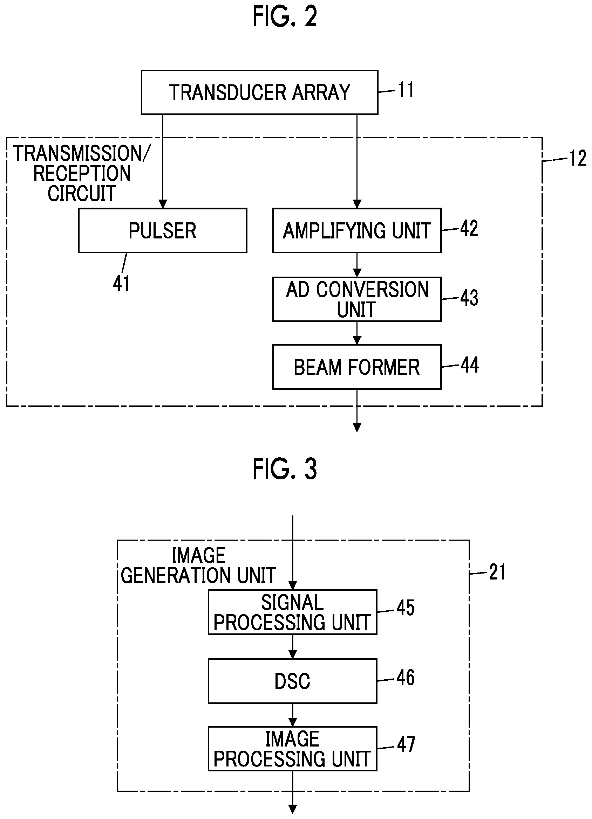

Under the control of the body controller 30, the transmission/reception circuit 12 causes the transducer array 11 to transmit the ultrasound and generates a sound ray signal based on a reception signal acquired by the transducer array 11. As shown in FIG. 2, the transmission/reception circuit 12 includes a pulser 41 connected to the transducer array 11, and an amplifying unit 42, an analog-to-digital (AD) conversion unit 43, and a beam former 44 that are sequentially connected in series to the transducer array 11.

The pulser 41 includes, for example, a plurality of pulse generators, and the pulser 41 adjusts an amount of delay of each drive signal such that the ultrasound transmitted from the plurality of ultrasound transducers of the transducer array 11 form the ultrasound beam, based on a transmission delay pattern selected in accordance with a control signal from the body controller 30, and supplies the adjusted signal to the plurality of ultrasound transducers. As described above, in a case in which a pulsed or continuous wave-like voltage is applied to the electrodes of the ultrasound transducer of the transducer array 11, the piezoelectric material expands and contracts to generate pulsed or continuous wave-like ultrasound from each of the ultrasound transducers, whereby the ultrasound beam is formed from the combined wave of the ultrasound.

The transmitted ultrasound beam is, for example, reflected by a target such as a part of the subject and propagates toward the transducer array 11 of the ultrasound probe 1. The ultrasound echo propagating toward the transducer array 11 as described above is received by each of the ultrasound transducers constituting the transducer array 11. In such a case, each of the ultrasound transducers constituting the transducer array 11 receives the propagating ultrasound echo to expand and contract, generates the reception signal, which is an electrical signal, and outputs these reception signals to the amplifying unit 42.

The amplifying unit 42 amplifies the signal input from each of the ultrasound transducers constituting the transducer array 11, and transmits the amplified signal to the AD conversion unit 43. The AD conversion unit 43 converts the signal, which is transmitted from the amplifying unit 42, into digital reception data. The beam former 44 performs so-called reception focus processing by applying respective delays to the reception data received from the AD conversion unit 43 and then adding the delayed data together. By the reception focus processing, each reception data, which is converted by the AD conversion unit 43, is phase-added, and the sound ray signal in which the focus of the ultrasound echo is narrowed down is acquired.

As shown in FIG. 3, the image generation unit 21 has a configuration in which a signal processing unit 45, a digital scan converter (DSC) 46, and an image processing unit 47 are sequentially connected in series.

The signal processing unit 45 generates a B-mode image signal, which is tomographic image information related to tissues inside the subject, by performing, on the sound ray signal received from the transmission/reception circuit 12, correction of the attenuation due to a distance in accordance with a depth of a reflection position of the ultrasound by using a sound velocity value set by the body controller 30 and then performing envelope detection processing.

The DSC 46 converts (raster-converts) the B-mode image signal, which is generated by the signal processing unit 45, into the image signal in accordance with a normal television signal scanning method.

The image processing unit 47 performs various types of necessary image processing such as gradation processing on the B-mode image signal input from the DSC 46, and then transmits the B-mode image signal to the display controller 22, the memory 24, the appropriateness calculation unit 25, and the feature extraction unit 26. Hereinafter, the B-mode image signal, which is image-processed by the image processing unit 47, will be referred to as an ultrasound image.

The ultrasound diagnostic apparatus according to the embodiment of the present invention is used to measure cardiac functions such as a so-called left ventricular ejection fraction (LVEF), a left ventricular end-diastolic volume, a left ventricular end-systolic volume, a left ventricular local wall motion, a global longitudinal strain (GLS), a mitral annular plane systolic excursion (MAPSE), a tricuspid annular plane systolic excursion (TAPSE), a cardiac output, and a single stroke volume.

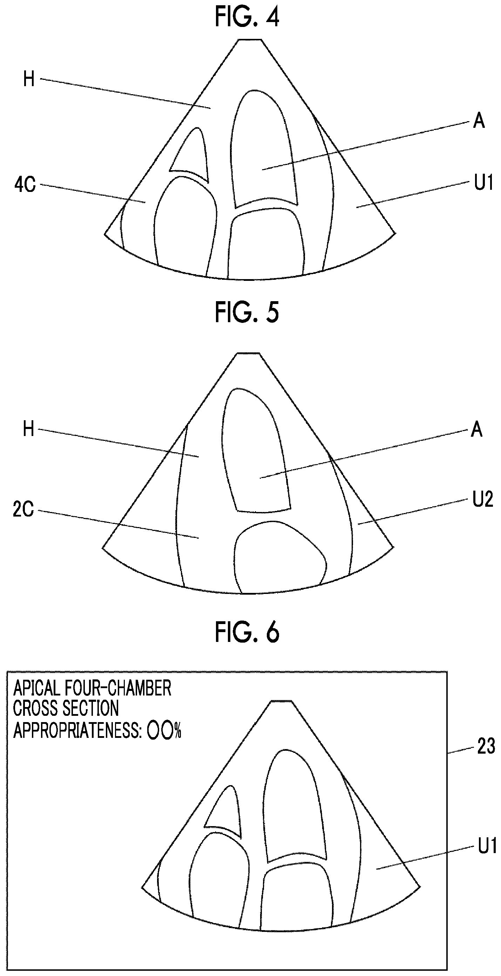

In order to measure the cardiac function, for example, as shown in FIGS. 4 and 5, the image acquisition unit 32 acquires first ultrasound images U1 of a plurality of frames representing a so-called apical four-chamber cross section 4C and the like of the heart H and second ultrasound images U2 of a plurality of frames representing a so-called apical two-chamber cross section 2C and the like. The first ultrasound image U1 and the second ultrasound image U2 is obtained by imaging the same cardiac chamber A and represent different cross sections of the cardiac chamber A. Here, the cardiac chamber A refers to any one of a left ventricle, a left atrium, a right ventricle, or a right atrium. In addition to the apical four-chamber cross section 4C and the apical two-chamber cross section 2C, for example, a so-called apical three-chamber cross section and a so-called apical five-chamber cross section, which are cross sections of the cardiac chamber A passing through the cardiac apex, can be imaged as the cross section of the cardiac chamber A.

The display controller 22 performs predetermined processing on the first ultrasound image U1, the second ultrasound image U2, and the like acquired by the image acquisition unit 32, to display the first ultrasound image U1, the second ultrasound image U2, and the like on the monitor 23 under the control of the body controller 30.

The monitor 23 displays the first ultrasound image U1, the second ultrasound image U2, and the like under the control of the display controller 22, and includes, for example, a display device such as a liquid crystal display (LCD) or an organic electroluminescence display (organic EL display).

The memory 24 stores the first ultrasound image U1, the second ultrasound image U2, and the like acquired by the image acquisition unit 32 under the control of the body controller 30.

In addition, as the memory 24, for example, a recording medium such as a flash memory, a hard disk drive (HDD), a solid-state drive (SSD), a flexible disk (FD), a magneto-optical disk (MO disk), a magnetic tape (MT), a random-access memory (RAM), a compact disc (CD), a digital versatile disc (DVD), a secure digital card (SD card), or a universal serial bus memory (USB memory) can be used.

The body controller 30 controls each unit of the apparatus body 2 and the transmission/reception circuit 12 of the ultrasound probe 1 based on a control program and the like stored in advance.

The input device 31 is an input device for the user to perform an input operation, and is configured by, for example, a device such as a keyboard, a mouse, a trackball, a touchpad, and a touch sensor disposed in a state of being superimposed on the monitor 23.

The appropriateness calculation unit 25 calculates the appropriateness as the target for measurement of the cardiac function for each of the first ultrasound image U1 and the second ultrasound image U2. The appropriateness, as the target for measurement of the cardiac function, of each of the first ultrasound image U1 and the second ultrasound image U2 refers to a degree to which a cross section represented by the first ultrasound image U1 and a cross section represented by the second ultrasound image U2 appropriately represent a predetermined cross section corresponding to the measurement of a specific cardiac function.

Specifically, as the appropriateness, a similarity between the cross section represented by the first ultrasound image U1 and a first cross section suitable for measuring the specific cardiac function, and a similarity between the cross section represented by the second ultrasound image U2 and a second cross section suitable for measuring the specific cardiac function can be used. As the cross section suitable for measuring the cardiac function, for example, a cross section of the cardiac chamber A which passes through the cardiac apex, such as the apical four-chamber cross section 4C, the apical two-chamber cross section 2C, the apical three-chamber cross section, and the apical five-chamber cross section and in which a long axis of the cardiac chamber A is maximized, is used. The long axis of the cardiac chamber A refers to a longest line segment that passes through the cardiac apex and that connects the cardiac apex to one point on the contour of the cardiac chamber A.

The appropriateness calculation unit 25 can store, for example, a plurality of measurement items of the cardiac function, such as a left ventricular ejection fraction and a cardiac output, and a type of a cross section suitable for each of the plurality of measurement items of the cardiac function, and calculate the appropriateness of the first ultrasound image U1 and the second ultrasound image U2 for the measurement item selected by the user via the input device 31. For example, in a case in which the left ventricular ejection fraction is selected as the measurement item, the appropriateness calculation unit 25 can calculate the appropriateness of the first ultrasound image U1 for the apical four-chamber cross section 4C and the appropriateness of the second ultrasound image U2 for the apical two-chamber cross section 2C.

In addition, in general, an ultrasound image representing a so-called parasternal left ventricular long-axis cross section and an ultrasound image representing an apical five-chamber cross section are often used for measurement in a case in which the cardiac output is measured, but in a case in which the ultrasound image representing the apical five-chamber cross section includes a protrusion or calcification of the interventricular septum, the cardiac output may not be normally measured. In this case, an ultrasound image representing the apical three-chamber cross section may be used instead of the ultrasound image representing the apical five-chamber cross section. Therefore, in a case in which the cardiac output is selected as the measurement item, the appropriateness calculation unit 25 can calculate, for example, the appropriateness of the first ultrasound image U1 for the apical five-chamber cross section and the appropriateness of the second ultrasound image U2 representing the apical three-chamber cross section.

For example, the appropriateness calculation unit 25 can store in advance a template image representing a typical image pattern of a plurality of cross sections corresponding to a plurality of measurement items of the cardiac function, calculate a similarity between the first ultrasound image U1 and a first template image corresponding to the first ultrasound image U1 as the appropriateness of the first ultrasound image U1, and calculate a similarity between the second ultrasound image U2 and a second template image corresponding to the second ultrasound image U2 as the appropriateness of the first ultrasound image U1. In addition, the appropriateness calculation unit 25 can calculate the appropriateness of the first ultrasound image U1 and the appropriateness of the second ultrasound image U2 using a learning model in so-called machine learning, which has learned a relationship between a cross section represented by a large number of ultrasound images obtained by imaging the cardiac chamber A, a plurality of cross sections corresponding to a plurality of measurement items of the cardiac function, and the similarity thereof, that is, the appropriateness of each ultrasound image for each cross section.

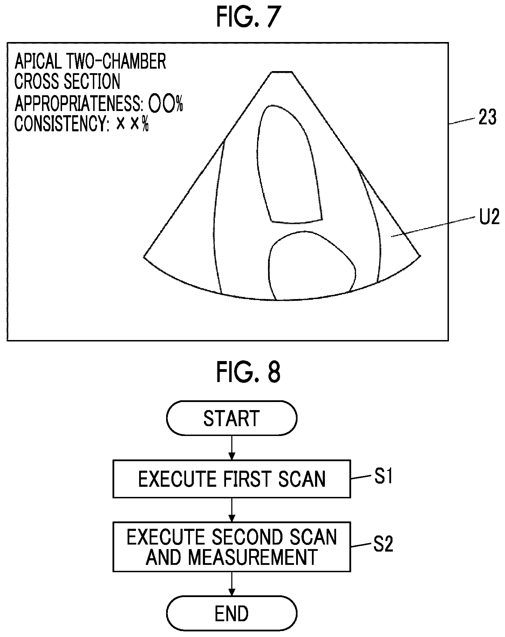

For example, the display controller 22 can sequentially display a value of the appropriateness together with the first ultrasound image U1 or the second ultrasound image U2 on the monitor 23 as shown in FIGS. 6 and 7 each time the appropriateness of the first ultrasound image U1 is calculated by the appropriateness calculation unit 25 and each time the appropriateness of the second ultrasound image U2 is calculated. The user can capture the first ultrasound image U1 and the second ultrasound image U2 by adjusting a position and an inclined angle of the ultrasound probe 1 while checking the value of the appropriateness displayed on the monitor 23.

The feature extraction unit 26 extracts a feature of an anatomical structure of the heart H from each of the first ultrasound image U1 and the second ultrasound image U2 acquired by the image acquisition unit 32. The feature extraction unit 26 can extract, for example, parameters such as a depth position of the cardiac apex and a length of the long axis of the cardiac chamber A, of which the values are ideally constant between cross sections and do not change with the cardiac beat, as the features of the anatomical structure of the heart H in the first ultrasound image U1 and the second ultrasound image U2, in the cross section of the cardiac chamber A passing through the cardiac apex, such as the apical four-chamber cross section 4C, the apical two-chamber cross section 2C, the apical three-chamber cross section, and the apical five-chamber cross section.

The consistency calculation unit 27 calculates consistency between the feature of the anatomical structure of the heart H in the first ultrasound image U1 extracted by the feature extraction unit 26 and the feature of the anatomical structure of the heart H in the second ultrasound image U2 extracted by the feature extraction unit 26. This consistency can be calculated, for example, as a value obtained by subtracting a rate of change in the feature of the anatomical structure in the second ultrasound image U2 from the anatomical structure in the first ultrasound image U1 from 100%.

For example, in a case in which a depth position P1 of the cardiac apex in the first ultrasound image U1 and a depth position P2 of the cardiac apex in the second ultrasound image U2 are extracted as the feature of the anatomical structure of the heart H in the first ultrasound image U1 and the feature of the anatomical structure of the heart H in the second ultrasound image U2, the consistency calculation unit 27 can calculate the consistency by [100% - (P1 - P2)/P1 × 100%] = P2/P1 × 100%. In addition, in a case in which a length L1 of the long axis of the cardiac chamber A in the first ultrasound image U1 and a length L2 of the long axis of the cardiac chamber A in the second ultrasound image U2 are extracted as the feature of the anatomical structure of the heart H in the first ultrasound image U1 and the feature of the anatomical structure of the heart H in the second ultrasound image U2, the consistency calculation unit 27 can calculate the consistency by [100% - (L1 - L2)/L1 × 100%] = L2/L1 × 100%.

Here, although the two different cross sections of the cardiac chamber A passing through the cardiac apex can be imaged by rotating the ultrasound probe 1 about an axis along a direction in which the ultrasound probe 1 is pressed against the body surface of the subject, the feature of the anatomical structure of the heart H extracted by the feature extraction unit 26 is ideally invariant with respect to the rotation of the ultrasound probe 1, and the consistency calculated by the consistency calculation unit 27 is ideally 100%. In a case in which the position or the inclined angle of the ultrasound probe 1 is changed in a case in which the ultrasound probe 1 is rotated to image another cross section from a state of imaging the specific cross section, the depth position of the cardiac apex and the length of the long axis of the cardiac chamber A are changed, and the consistency is decreased from 100%. As described above, the consistency can be understood as an indicator indicating a rate of match of the position and the inclined angle of the ultrasound probe 1 between the time of capturing the first ultrasound image U1 and the time of capturing the second ultrasound image U2.

The measurement instruction unit 28 has a predetermined consistency threshold value, such as 90%, for the consistency, and determines whether or not the consistency calculated by the consistency calculation unit 27 between the first ultrasound image U1 and the second ultrasound image U2 is equal to or greater than the consistency threshold value. In addition, the measurement instruction unit 28 has a predetermined appropriateness threshold value for the appropriateness, and determines whether or not the appropriateness of each of the first ultrasound image U1 and the second ultrasound image U2 is equal to or greater than the appropriateness threshold value. In a case in which the consistency calculated between the first ultrasound image U1 and the second ultrasound image U2 is equal to or greater than the consistency threshold value and the appropriateness calculated for each of the first ultrasound image U1 and the second ultrasound image U2 is equal to or greater than the appropriateness threshold value, the measurement instruction unit 28 instructs the measurement unit 29 to execute the measurement of the cardiac function. As a result, since the measurement can be executed using the first ultrasound image U1 and the second ultrasound image U2 suitable for measuring the cardiac function, the cardiac function can be accurately measured.

The measurement unit 29 measures the cardiac function of the subject based on the first ultrasound image U1 and the second ultrasound image U2. In a case of measuring, for example, the left ventricular ejection fraction as the cardiac function, the measurement unit 29 extracts a left ventricle shown in the first ultrasound image U1 by performing image analysis of the first ultrasound image U1 representing the apical four-chamber cross section 4C, and calculates a volume of the extracted left ventricle in the first ultrasound image U1. In addition, the measurement unit 29 extracts a left ventricle shown in the second ultrasound image U2 representing the apical two-chamber cross section 2C, and calculates a volume thereof in the same manner. The measurement unit 29 can measure the left ventricular ejection fraction based on the volume of the left ventricle in the first ultrasound image U1 and the volume of the left ventricle in the second ultrasound image U2 calculated as described above. In this case, the measurement unit 29 can calculate the left ventricular ejection fraction by calculating the volume using, for example, a so-called modified Simpson method, a stacked-disk method, an area-length method, and the like.

The measurement unit 29 can extract the left ventricle from the first ultrasound image U1 and the second ultrasound image U2 by a so-called template matching method of searching for the inside of the first ultrasound image U1 and the inside of the second ultrasound image U2 using, for example, a template image representing a typical image pattern of the left ventricle. In addition, the measurement unit 29 can also extract the left ventricle from the first ultrasound image U1 and the second ultrasound image U2 using, for example, a trained model in machine learning, which has learned a relationship between a large number of ultrasound images and the left ventricle shown in the ultrasound images.

In a case of measuring, for example, the cardiac output as the cardiac function, the measurement unit 29 extracts an aorta shown in the ultrasound image by performing image analysis of the ultrasound image representing the parasternal left ventricular long-axis cross section, and calculates a diameter of the extracted aorta. The measurement unit 29 extracts the aorta shown in the first ultrasound image U1 by performing image analysis of the first ultrasound image U1 representing the apical five-chamber cross section, and installs a so-called Doppler gate on the extracted aorta in the first ultrasound image U1. The measurement unit 29 calculates a blood flow velocity in the aorta at a position of the Doppler gate using a so-called pulse Doppler method. Further, the measurement unit 29 can measure the cardiac output based on the calculated diameter of the aorta and the calculated blood flow velocity in the aorta.

In the first ultrasound image U1 representing the apical five-chamber cross section, in a case in which the interventricular septum protrudes to overlap the position of the aorta, in a case in which there is calcification in the aorta, or the like, the blood flow velocity cannot be accurately measured, so that the measurement unit 29 can calculate the blood flow velocity in the aorta using, for example, the second ultrasound image U2 representing the apical three-chamber cross section instead of the first ultrasound image U1 representing the apical five-chamber cross section.

In the present embodiment, each processing is executed by any computer. In addition, any computer may execute these pieces of processing by a processor as hardware, a program as software, or a combination thereof. In such a case, the processor is configured to execute various types of processing in the present embodiment in cooperation with the program, and can function as each unit or each means in the present embodiment. In addition, the execution order of the processing by the processor is not limited to the order described above and may be changed as appropriate. Any computer may be a general-purpose computer, a computer for a specific use, a workstation, or another system capable of executing each processing.

The processor 33 including the image generation unit 21, the display controller 22, the appropriateness calculation unit 25, the feature extraction unit 26, the consistency calculation unit 27, the measurement instruction unit 28, the measurement unit 29, and the body controller 30 may be configured by one or a plurality of pieces of hardware, and the type of hardware is not limited. For example, the processor 33 can be configured by a programmable logic device such as a central processing unit (CPU), a micro processing unit (MPU), or a field programmable gate array (FPGA), a dedicated circuit for executing specific processing, such as an application specific integrated circuit (ASIC), or hardware such as a graphic processing unit (GPU) or a neural processing unit (NPU). The types of hardware may be a combination of different types of hardware. In a case in which a plurality of types of hardware are configured to execute one or a plurality of types of processing of a certain processor, the plurality of types of hardware may be present in devices physically separated from each other or may be present in the same device. In any of the embodiments, the order of each processing performed by the processor is not limited to the above-described order, and may be changed as appropriate. Hardware is implemented in a form of an electric circuit (circuitry) in which circuit elements such as semiconductor elements are combined.

Further, the program may be software, such as firmware or a microcode. Further, the program may be, for example, a program module group, and each function thereof may be implemented by a processor configured to execute each function. The program may be a program code or a plurality of code segments stored in one or a plurality of non-transitory computer-readable media (for example, a storage medium or another storage). The program may be stored in a plurality of non-transitory computer-readable media present in devices physically separated from each other. The program code or the code segment can represent any combination of procedures, functions, subprograms, routines, subroutines, modules, software packages, classes, instructions, data structures, or program statements. The program code or the code segment may be connected to another code segment or a hardware circuit by transmitting and receiving information, data, arguments, parameters, or contents of the memory.

Hereinafter, an operation of the ultrasound diagnostic apparatus according to Embodiment will be described with reference to a flowchart shown in FIG. 8. Here, an example will be described in which the left ventricular ejection fraction is calculated as the cardiac function, but the present invention is not limited to a case in which the left ventricular ejection fraction is calculated as the cardiac function, and can also be applied to a case in which other cardiac functions, such as the cardiac output, are calculated, for example, based on the ultrasound image representing the cross section of the heart H passing through the cardiac apex.

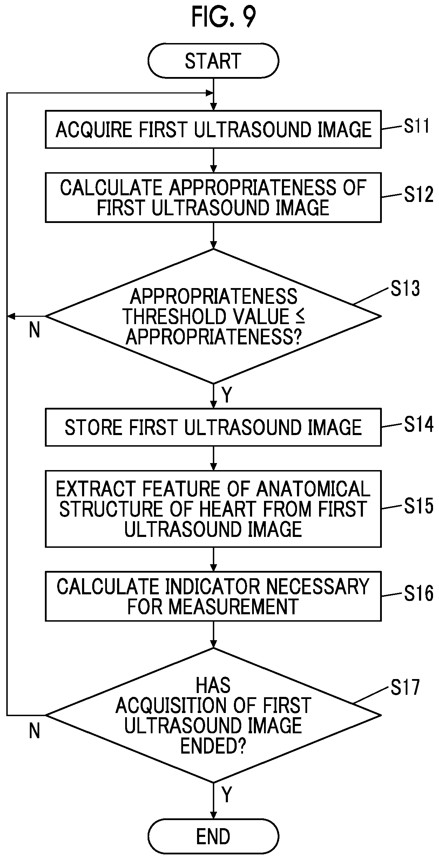

In step S1, a first scan for acquiring the first ultrasound images U1 of the plurality of frames is performed. In the first scan, the volume of the left ventricle in the first ultrasound image U1 at the end-diastolic phase of the heart H and the volume of the left ventricle in the first ultrasound image U1 at the end-systolic phase of the heart H are acquired as indicators necessary for measuring the left ventricular ejection fraction from the first ultrasound image U1 suitable for measuring the left ventricular ejection fraction. Here, the first ultrasound image U1 suitable for measuring the left ventricular ejection fraction refers to the first ultrasound image U1 which represents the apical four-chamber cross section 4C as shown in FIG. 4 and in which the length of the long axis of the left ventricle is maximized. The processing of step S1 includes a plurality of steps in the flowchart shown in FIG. 9.

First, in step S11, the user disposes the ultrasound probe 1 at the position for capturing the first ultrasound image U1 representing the apical four-chamber cross section 4C as shown in FIG. 4. The image acquisition unit 32 acquires the first ultrasound image U1 representing the cross section of the left ventricle. In such a case, under the control of the body controller 30, the transmission and reception of the ultrasound from the plurality of transducers of the transducer array 11 are started in accordance with the drive signal from the pulser 41 of the transmission/reception circuit 12 of the ultrasound probe 1, the ultrasound echo from the subject is received by the plurality of transducers of the transducer array 11, and the reception signal as the analog signal is output to the amplifying unit 42, is amplified, and then is subjected to the AD conversion via the AD conversion unit 43 to acquire the reception data.

The reception focus processing is performed on the reception data by the beam former 44, the sound ray signal generated by the reception focusing processing is transmitted to the image generation unit 21 of the apparatus body 2, and thus the first ultrasound image U1 is generated by the image generation unit 21. In this case, the signal processing unit 45 of the image generation unit 21 performs the correction of the attenuation in accordance with the depth of the reflection position of the ultrasound and the envelope detection processing on the sound ray signal, the DSC 46 performs the conversion into the image signal in accordance with the normal television signal scanning method, and the image processing unit 47 performs various types of necessary image processing, such as gradation processing. The first ultrasound image U1 generated in step S11 in this way is transmitted to the display controller 22, the memory 24, the appropriateness calculation unit 25, and the feature extraction unit 26.

In step S12, the appropriateness calculation unit 25 calculates the appropriateness of the first ultrasound image U1 acquired in step S11 as the target for measurement of the left ventricular ejection fraction. The appropriateness calculation unit 25 can calculate the similarity between the template image representing the cross section which is the apical four-chamber cross section 4C as shown in FIG. 4 and in which the length of the long axis of the left ventricle is maximized and the first ultrasound image U1, as the appropriateness of the first ultrasound image U1. In a case in which the appropriateness is calculated in step S12, the display controller 22 displays the calculated value of the appropriateness on the monitor 23 together with the first ultrasound image U1, for example, as shown in FIG. 6.

In step S13, the measurement instruction unit 28 determines whether or not the appropriateness of the first ultrasound image U1 calculated in step S12 is equal to or greater than the predetermined appropriateness threshold value. While it is determined in step S13 that the appropriateness is less than the appropriateness threshold value, the processing of step S11 to step S13 is repeated. In a case in which it is determined in step S13 that the appropriateness is equal to or greater than the appropriateness threshold value, the appropriateness is stored in the memory 24, and the processing proceeds to step S14.

In step S14, the memory 24 stores a latest first ultrasound image U1 acquired in step S11 under the control of the body controller 30.

In step S15, the feature extraction unit 26 extracts the feature of the anatomical structure of the heart H from the first ultrasound image U1 by performing image analysis of the latest first ultrasound image U1 acquired in step S11. The feature extraction unit 26 can extract, for example, the depth position of the cardiac apex or the length of the long axis of the left ventricle in the first ultrasound image U1 as the feature of the anatomical structure of the heart H.

In step S16, the measurement unit 29 calculates, as the indicator necessary for measuring the left ventricular ejection fraction, the volume of the left ventricle in the first ultrasound image U1 at the end-diastolic phase of the heart H and a volume of the left ventricle in the first ultrasound image U1 at the end-systolic phase of the heart H by analyzing the first ultrasound image U1 stored in the memory 24 in step S14.

In this case, the measurement unit 29 extracts the left ventricle shown in the first ultrasound image U1 by performing image analysis of the first ultrasound image U1 stored in the memory 24 in step S14, using a method of template matching or a method of using a trained model in machine learning, calculates the area in the first ultrasound image U1 by counting the total number of pixels surrounded by the contour of the extracted left ventricle, and calculates the volume of the left ventricle from the calculated area.

The measurement unit 29 can determine whether or not the first ultrasound image U1 represents any of the left ventricle at the end-diastolic phase or the left ventricle at the end-systolic phase, for example, based on a change in the volume of the left ventricle in the first ultrasound images U1 of the plurality of frames consecutive in time series. At the current point in time, only the first ultrasound image U1 of one frame is stored in the memory 24, and thus it is not possible to perform this determination. In this way, in a case in which it is not possible to determine whether the first ultrasound image U1 represents any of the left ventricle at the end-diastolic phase or the left ventricle at the end-systolic phase, step S16 is skipped.

In step S17, the body controller 30 determines whether or not to end the acquisition of the first ultrasound image U1. The body controller 30 can determine to end the acquisition of the first ultrasound image U1, for example, in a case in which the user gives an instruction to end the acquisition of the first ultrasound image U1 via the input device 31. In addition, in a case in which the instruction to end the acquisition of the first ultrasound image U1 is not particularly input by the user via, for example, the input device 31, the body controller 30 can determine to continue the acquisition of the first ultrasound image U1.

While it is determined in step S17 to continue the acquisition of the first ultrasound image U1, the processing of step S11 to step S17 is repeated. As a result, the first ultrasound images U1 of the plurality of frames are stored in the memory 24 in step S14. In addition, the feature of the anatomical structure of the heart H, such as the depth position of the cardiac apex and the length of the long axis of the left ventricle, should ideally not change in the first ultrasound images U1 of the plurality of frames. Therefore, in step S15, only the initially extracted feature of the anatomical structure of the heart H is stored in the memory 24, and the subsequent processing can be skipped. In addition, in step S15, an average value of the features of the anatomical structure of the heart H extracted from the first ultrasound images U1 of the plurality of frames can be calculated, and the calculated average value can be stored in the memory 24 as the feature of the anatomical structure of the heart H.

Further, in step S16, the measurement unit 29 can create and update time-series change data of the volume of the left ventricle by arranging the calculated volumes of the left ventricle in the first ultrasound image U1 in time series, each time the processing of step S11 to step S17 is repeated, acquire the maximum value in the updated time-series change data as the volume of the left ventricle at the end-diastolic phase, and acquire the minimum value in the time-series change data as the volume of the left ventricle at the end-systolic phase.

In a case in which the user determines that the indicators necessary for measuring the left ventricular ejection fraction are sufficiently calculated and it is determined to end the acquisition of the first ultrasound image U1 in step S17 based on the instruction from the user, the processing of the first scan in step S1 according to the flowchart of FIG. 9 is completed.

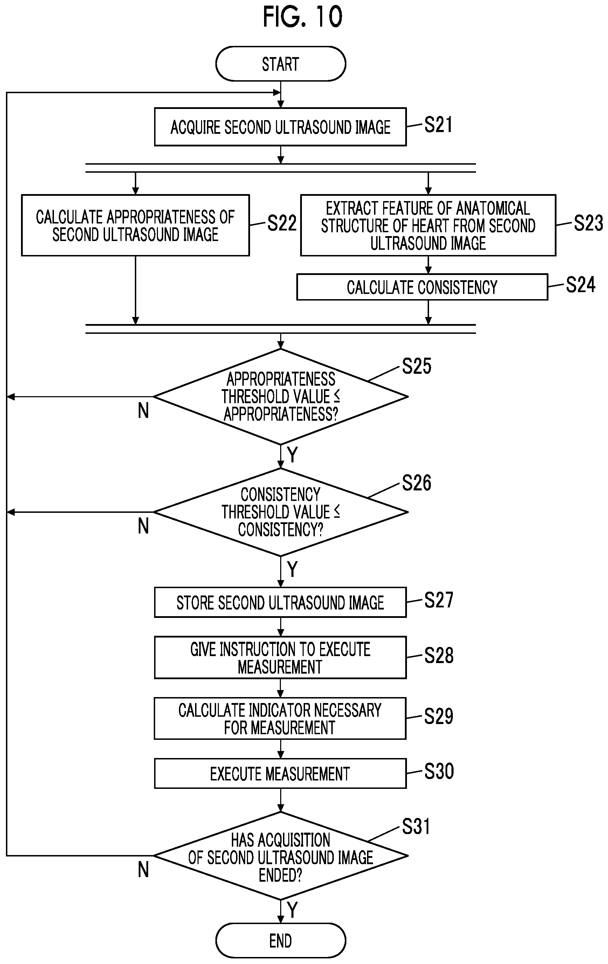

In step S2 following step S1, a second scan for acquiring a plurality of second ultrasound images U2 and the measurement of the left ventricular ejection fraction during the second scan are performed. In the second scan, the volume of the left ventricle in the second ultrasound image U2 at the end-diastolic phase of the heart H and the volume of the left ventricle in the second ultrasound image U2 at the end-systolic phase of the heart H are acquired as the indicators necessary for measuring the left ventricular ejection fraction from the second ultrasound image U2 suitable for measuring the left ventricular ejection fraction, and the left ventricular ejection fraction is measured based on the indicators acquired in the first scan in step S1 and the indicators acquired in the second scan in step S2. Here, the second ultrasound image U2 suitable for measuring the left ventricular ejection fraction refers to the second ultrasound image U2 which represents the apical two-chamber cross section 2C as shown in FIG. 5 and in which the length of the long axis of the left ventricle is maximized. The processing of step S2 includes a plurality of steps in the flowchart shown in FIG. 10.

First, in step S21, the user disposes the ultrasound probe 1 at the position for capturing the second ultrasound image U2 representing the apical two-chamber cross section 2C as shown in FIG. 5. The image acquisition unit 32 acquires the second ultrasound image U2 representing the cross section of the left ventricle by the same method as in step S11. The second ultrasound image U2 generated in step S11 in this way is transmitted to the display controller 22, the memory 24, the appropriateness calculation unit 25, and the feature extraction unit 26. In a case in which the processing of step S21 is completed in this way, the processing of step S22 and the processing of step S23 and step S24 are performed in parallel.

In step S22, the appropriateness calculation unit 25 calculates the appropriateness of the second ultrasound image U2 acquired in step S21 as the target for measurement of the left ventricular ejection fraction by the same method as in step S12. The appropriateness calculation unit 25 can calculate the similarity between the template image representing the cross section which is the apical two-chamber cross section 2C as shown in FIG. 5 and in which the length of the long axis of the left ventricle is maximized and the second ultrasound image U2, as the appropriateness of the second ultrasound image U2.

In step S23, the feature extraction unit 26 extracts the feature of the same anatomical structure as the anatomical structure of the heart H extracted from the first ultrasound image U1 in step S15 from the second ultrasound image U2 by performing image analysis of the second ultrasound image U2 acquired in step S21.

In step S24, the consistency calculation unit 27 calculates the consistency between the feature of the anatomical structure in the first ultrasound image U1, which is extracted in step S15 and stored in the memory 24, and the feature of the anatomical structure in the second ultrasound image U2 extracted in step S23. For example, the consistency calculation unit 27 can calculate a value obtained by subtracting the rate of change in the feature of the anatomical structure in the second ultrasound image U2 from the feature of the anatomical structure in the first ultrasound image U1 from 100% as the consistency.

In a case in which the appropriateness of the second ultrasound image U2 is calculated in step S22 and the consistency is calculated in step S24, for example, as shown in FIG. 7, these values are displayed on the monitor 23 together with the second ultrasound image U2 acquired in step S21.

In subsequent step S25, the measurement instruction unit 28 determines whether or not the appropriateness of the second ultrasound image U2 calculated in step S22 is equal to or greater than the predetermined appropriateness threshold value. While it is determined in step S25 that the appropriateness is less than the appropriateness threshold value, the processing of step S21 to step S25 is repeated. In a case in which it is determined in step S25 that the appropriateness is equal to or greater than the appropriateness threshold value, the processing proceeds to step S26.

In step S26, the measurement instruction unit 28 determines whether or not the consistency calculated in step S24 is equal to or greater than the predetermined consistency threshold value. While it is determined in step S26 that the consistency is less than the consistency threshold value, the processing of step S21 to step S26 is repeated. In a case in which it is determined in step S26 that the consistency is equal to or greater than the consistency threshold value, the processing proceeds to step S27.

In step S27, the memory 24 stores the latest second ultrasound image U2 acquired in step S21 under the control of the body controller 30. In this way, the second ultrasound image U2, which represents the apical two-chamber cross section 2C appropriate as the target for measurement of the left ventricular ejection fraction and which is captured by the ultrasound probe 1 at the same position and inclined angle as in the capturing of the first ultrasound image U1, is automatically stored.

In step S28, the measurement instruction unit 28 instructs the measurement unit 29 to measure the left ventricular ejection fraction.

In step S29, the measurement unit 29 calculates, as the indicator necessary for measuring the left ventricular ejection fraction, the volume of the left ventricle in the second ultrasound image U2 at the end-diastolic phase of the heart H and a volume of the left ventricle in the second ultrasound image U2 at the end-systolic phase of the heart H by analyzing the second ultrasound image U2 stored in the memory 24 in step S27.

In this case, the measurement unit 29 extracts the left ventricle shown in the second ultrasound image U2 by performing image analysis of the second ultrasound image U2 stored in the memory 24 in step S27, using a method of template matching or a method of using a trained model in machine learning, calculates the area in the second ultrasound image U2 by counting the total number of pixels surrounded by the contour of the extracted left ventricle, and calculates the volume of the left ventricle from the calculated area.

The measurement unit 29 can determine whether or not the second ultrasound image U2 represents any of the left ventricle at the end-diastolic phase or the left ventricle at the end-systolic phase, for example, based on a change in the volume of the left ventricle in the second ultrasound image U2 of the plurality of frames consecutive in time series. At the current point in time, only the second ultrasound image U2 of one frame is stored in the memory 24, and thus it is not possible to perform this determination. In this way, in a case in which it is not possible to determine whether the second ultrasound image U2 represents any of the left ventricle at the end-diastolic phase or the left ventricle at the end-systolic phase, step S28 is skipped.

In step S30, the measurement unit 29 measures the left ventricular ejection fraction using the indicator necessary for the measurement calculated in step S1 and the indicator necessary for the measurement calculated in step S29. At the current point in time, since the processing of step S28 is skipped, the processing of step S30 is also skipped.

In step S31, the body controller 30 determines whether or not to end the acquisition of the second ultrasound image U2. The body controller 30 can determine to end the acquisition of the second ultrasound image U2, for example, in a case in which the user gives an instruction to end the acquisition of the second ultrasound image U2 via the input device 31. In addition, in a case in which the instruction to end the acquisition of the second ultrasound image U2 is not particularly input by the user via, for example, the input device 31, the body controller 30 can determine to continue the acquisition of the second ultrasound image U2.

While it is determined in step S31 to continue the acquisition of the second ultrasound image U2, the processing of step S21 to step S31 is repeated. As a result, the second ultrasound images U2 of the plurality of frames are stored in the memory 24 in step S27. Further, in step S29, the measurement unit 29 can create and update time-series change data of the volume of the left ventricle by arranging the calculated volumes of the left ventricle in the second ultrasound image U2 in time series, each time the processing of step S21 to step S31 is repeated, acquire the maximum value in the updated time-series change data as the volume of the left ventricle at the end-diastolic phase, and acquire the minimum value in the time-series change data as the volume of the left ventricle at the end-systolic phase.

In step S29, in a case in which the indicator necessary for measuring the left ventricular ejection fraction is acquired from the second ultrasound image U2, the measurement unit 29 calculates the left ventricular ejection fraction by using this indicator and the indicator acquired from the first ultrasound image U1 in step S1. In this case, the measurement unit 29 can calculate a final volume of the left ventricle at the end-diastolic phase based on the volumes of the left ventricle in the first ultrasound image U1 and the second ultrasound image U2 corresponding to the end-diastolic phase of the heart H, calculate a final volume of the left ventricle at the end-systolic phase based on the volumes of the left ventricle in the first ultrasound image U1 and the second ultrasound image U2 corresponding to the end-systolic phase of the heart H, and calculate the left ventricular ejection fraction based on the calculated final volume of the left ventricle at the end-diastolic phase and the calculated final volume of the left ventricle at the end-systolic phase.

The measurement unit 29 displays the calculated value of the left ventricular ejection fraction on the monitor 23 via the display controller 22. In a case in which the maximum value or the minimum value of the volume of the left ventricle is updated in step S29 while the processing of step S21 to step S31 is repeated, the left ventricular ejection fraction is measured again using the maximum value or the minimum value of the volume of the left ventricle after the update in step S30, and the measured value obtained in this way is displayed on the monitor 23. In this way, during the second scan, the left ventricular ejection fraction is measured in real time, and the measured value is displayed on the monitor 23 from time to time.

Since both the plurality of first ultrasound images U1 and the plurality of second ultrasound images U2 represent the cross sections appropriate as the target for measurement and have sufficient consistency with each other, both the first ultrasound image U1 of the target for measurement and the second ultrasound image U2 of the target for measurement extracted by the measurement unit 29 are appropriate as the ultrasound images used for measurement. Therefore, the measurement unit 29 can accurately calculate the left ventricular ejection fraction based on the extracted first ultrasound image U1 of the target for measurement and the second ultrasound image U2 of the target for measurement.

In a case in which the user determines that the measurement of the left ventricular ejection fraction is sufficiently performed and it is determined to end the acquisition of the second ultrasound image U2 in step S31 based on the instruction from the user, the second scan in step S2 and the processing of measuring the left ventricular ejection fraction during the second scan are completed according to the flowchart of FIG. 10.

In a case in which the processing of step S2 is completed in this manner, the operation of the ultrasound diagnostic apparatus of Embodiment 1 according to the flowchart of FIG. 8 is completed.

As described above, with the ultrasound diagnostic apparatus according to Embodiment 1, the appropriateness calculation unit 25 calculates the appropriateness as the target for measurement for the first ultrasound image U1 and the second ultrasound image U2, the feature extraction unit 26 extracts the feature of the anatomical structure of the heart H from each of the first ultrasound image U1 and the second ultrasound image U2, the consistency calculation unit 27 calculates the consistency between the feature of the anatomical structure of the heart H in the first ultrasound image U1 and the feature of the anatomical structure of the heart H in the second ultrasound image U2, and the measurement instruction unit 28 instructs the measurement unit 29 to execute the measurement of the cardiac function in a case in which the calculated consistency is equal to or greater than the predetermined consistency threshold value and both the calculated appropriateness calculated for the first ultrasound image U1 and the calculated appropriateness calculated for the second ultrasound image U2 are equal to or greater than the predetermined appropriateness threshold value, so that it is possible to accurately perform the measurement of the cardiac function using the first ultrasound image U1 and the second ultrasound image U2.

A case has been described in which the transmission/reception circuit 12 is provided in the ultrasound probe 1, but the transmission/reception circuit 12 may be provided in the apparatus body 2.

Further, a case has been described in which the image generation unit 21 is provided in the apparatus body 2, but the image generation unit 21 may be provided in the ultrasound probe 1.

The apparatus body 2 may be a so-called stationary type, a portable type that is easily carried, or a so-called handheld type that is configured by, for example, a smartphone or a tablet type computer. In this way, the type of the device constituting the apparatus body 2 is not particularly limited.

Examples of the measurement items of the cardiac function using two types of ultrasound images, that is, the first ultrasound image U1 and the second ultrasound image U2 include the left ventricular ejection fraction using the ultrasound image representing the apical four-chamber cross section 4C and the ultrasound image representing the apical two-chamber cross section 2C, but other examples thereof include the left ventricular end-diastolic volume using the ultrasound image representing the apical two-chamber cross section 2C and the ultrasound image representing the apical four-chamber cross section 4C, the left ventricular end-systolic volume using the ultrasound image representing the apical two-chamber cross section 2C and the ultrasound image representing the apical four-chamber cross section 4C, and the like.

In addition, the present invention can also be applied to the measurement of the cardiac function using a plurality of types of ultrasound images representing three or more types of cross sections. In this case, the appropriateness calculation unit 25 can calculate the appropriateness as the target for measurement for each of the plurality of types of ultrasound images, the feature extraction unit 26 can extract the feature of the anatomical structure of the heart H from each of the plurality of types of ultrasound images, and the consistency calculation unit 27 can calculate the consistency between the features of the anatomical structure of the heart H in the plurality of types of ultrasound images. In this case, for example, the consistency calculation unit 27 can calculate the consistency between any of the feature of the anatomical structure of the heart H extracted from the first ultrasound image U1 or the feature of the anatomical structure of the heart H extracted from the second ultrasound image U2 and a third ultrasound image representing a cross section different from the cross sections in the first ultrasound image U1 and the second ultrasound image U2. In a case in which the consistency calculated between the plurality of types of ultrasound images is equal to or greater than the predetermined consistency threshold value and the appropriateness calculated for the plurality of types of ultrasound images is equal to or greater than the predetermined appropriateness threshold value, the measurement instruction unit 28 can instruct the measurement unit 29 to execute the measurement.

For example, examples of all the measurement items of the cardiac function using the ultrasound images representing three types of cross sections include left ventricular local wall motion, a GLS, an MAPSE, and the like using ultrasound images representing the apical two-chamber cross section 2C, the apical three-chamber cross section, and the apical four-chamber cross section 4C.

In addition, in a case in which different measurement items of the cardiac function are consecutively measured, the consistency calculation unit 27 can calculate the consistency between the feature of the anatomical structure of the heart H in the ultrasound image used for the measurement item that has been just completed and the feature of the anatomical structure of the heart H in the first ultrasound image U1 used for the current measurement item. In a case in which the appropriateness calculated for the first ultrasound image U1 to be used for the current measurement item is equal to or greater than the predetermined appropriateness and the consistency calculated for the first ultrasound image U1 to be used for the current measurement item is equal to or greater than the predetermined consistency threshold value, the measurement instruction unit 28 can store the first ultrasound image U1 in the memory 24 as a candidate for the first ultrasound image U1 to be actually used for the measurement. As described above, by taking into account the appropriateness of the first ultrasound image U1 used for the current measurement item and the consistency between the feature of the anatomical structure of the heart H in the ultrasound image used for the previous measurement item and the feature of the anatomical structure of the heart H in the first ultrasound image U1 used for the current measurement item, it is possible to obtain the first ultrasound image U1 that more appropriately represents the cross section of the target for measurement.

### Embodiment 2

Depending on the scanning position of the ultrasound probe 1, the posture of the subject, and a breathing state of the subject, the appropriateness of the first ultrasound image U1 and the second ultrasound image U2 may be less than the appropriateness threshold value, or the consistency between the feature of the anatomical structure of the heart H in the first ultrasound image U1 and the feature of the anatomical structure of the heart H in the second ultrasound image U2 may be less than the consistency threshold value. In such a case, the processor 33 can instruct the user to improve a cause thereof.

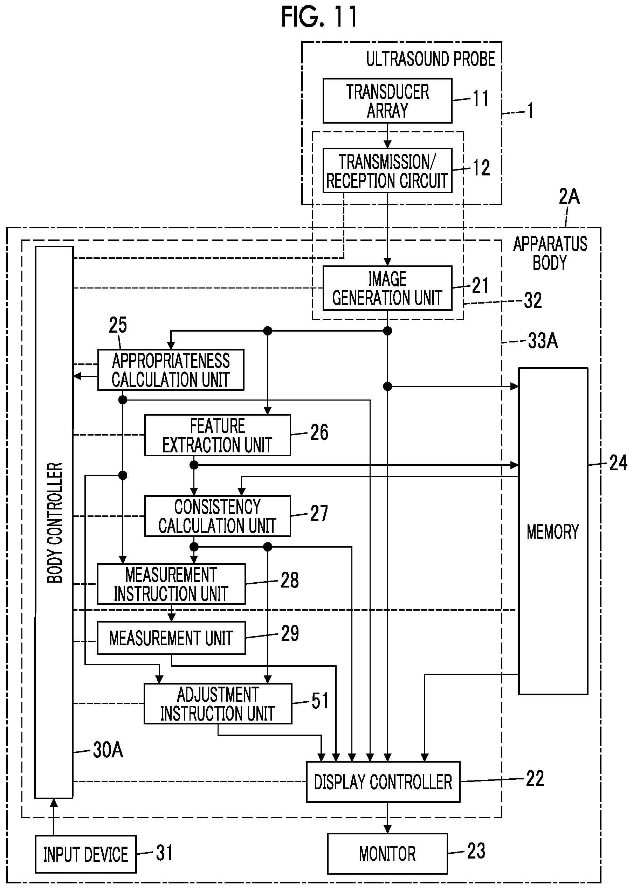

FIG. 11 shows a configuration of an ultrasound diagnostic apparatus of Embodiment 2. The ultrasound diagnostic apparatus according to Embodiment 2 comprises an apparatus body 2A instead of the apparatus body 2, as compared with the ultrasound diagnostic apparatus according to Embodiment 1 shown in FIG. 1. The apparatus body 2A in Embodiment 2 further comprises an adjustment instruction unit 51 and comprises a body controller 30A instead of the body controller 30, as compared with the apparatus body 2 in Embodiment 1.

In the apparatus body 2A, the adjustment instruction unit 51 is connected to the appropriateness calculation unit 25 and the consistency calculation unit 27. The adjustment instruction unit 51 is connected to the display controller 22 and the body controller 30A. In addition, the image generation unit 21, the display controller 22, the appropriateness calculation unit 25, the feature extraction unit 26, the consistency calculation unit 27, the measurement instruction unit 28, the measurement unit 29, the body controller 30A, and the adjustment instruction unit 51 constitute a processor 33A for the apparatus body 2A.

The adjustment instruction unit 51 instructs the user to adjust at least one of a scanning position of the ultrasound probe 1, a posture of the subject, or a breathing method of the subject in a case in which, for a predetermined time, the consistency calculated by the consistency calculation unit 27 between the first ultrasound image U1 and the second ultrasound image U2 is less than the predetermined consistency threshold value or any of the appropriateness calculated by the appropriateness calculation unit 25 for the first ultrasound image U1 or the second ultrasound image U2 is less than the predetermined appropriateness threshold value. For example, the adjustment instruction unit 51 can display a message indicating that at least one of the scanning position of the ultrasound probe 1, the posture of the subject, or the breathing method of the subject is to be adjusted, on the monitor 23.

With the ultrasound diagnostic apparatus according to Embodiment 2, the user can easily obtain the first ultrasound image U1 representing the cross section appropriate as the target for measurement or the second ultrasound image U2 representing the cross section appropriate as the target for measurement, or the second ultrasound image U2 showing the feature of the anatomical structure that is consistent with the feature of the anatomical structure of the heart H in the first ultrasound image U1 by adjusting at least one of the scanning position of the ultrasound probe 1, the posture of the subject, or the breathing method of the subject by checking the instruction from the adjustment instruction unit 51 even in a case in which the user has a low skill level of measurement, for example.

It should be noted that the example has been described in which the adjustment instruction unit 51 displays a message on the monitor 23 to issue an instruction to the user, but the method of issuing the instruction to the adjustment instruction unit 51 is not particularly limited to this. In a case in which the ultrasound diagnostic apparatus comprises a speaker (not shown), the adjustment instruction unit 51 can give an instruction to the user by, for example, sound via the speaker. In addition, in a case in which the ultrasound diagnostic apparatus comprises a lamp (not shown), the adjustment instruction unit 51 can give an instruction to the user by, for example, changing a light emission pattern in the lamp in accordance with an instruction content.

### Explanation of References

1: ultrasound probe
2, 2A: apparatus body
11: transducer array
12: transmission/reception circuit
21: image generation unit
22: display controller
23: monitor
24: memory
25: appropriateness calculation unit
26: feature extraction unit
27: consistency calculation unit
28: measurement instruction unit
29: measurement unit
30, 30A: body controller
31: input device
32: image acquisition unit
33, 33A: processor
41: pulser
42: amplifying unit
43: AD conversion unit
44: beam former
45: signal processing unit
46: DSC
47: image processing unit
51: adjustment instruction unit
2C: apical two-chamber cross section
4C: apical four-chamber cross section
A: cardiac chamber
H: heart
U1: first ultrasound image
U2: second ultrasound image

## Claims

1. An ultrasound diagnostic apparatus comprising:
an ultrasound probe (1);
a monitor (23);
an image acquisition unit (32) that acquires first and second ultrasound images in which different cross sections of a heart of a subject are imaged, by transmitting and receiving ultrasound beams using the ultrasound probe (1);
a feature extraction unit (26) that extracts a feature of an anatomical structure of the heart from each of the first ultrasound image and the second ultrasound image that are acquired by the image acquisition unit (32);
a consistency calculation unit (27) that calculates consistency between the feature of the anatomical structure in the first ultrasound image extracted by the feature extraction unit (26) and the feature of the anatomical structure in the second ultrasound image extracted by the feature extraction unit (26);
an appropriateness calculation unit (25) that calculates appropriateness as a target for measurement for each of the first ultrasound image and the second ultrasound image;
a measurement unit (29) that measures a cardiac function of the subject based on the first ultrasound image and the second ultrasound image; and
a measurement instruction unit (28) that instructs the measurement unit (29) to execute measurement in a case in which the consistency calculated by the consistency calculation unit (27) between the first ultrasound image and the second ultrasound image is equal to or greater than a predetermined consistency threshold value, and both the appropriateness calculated by the appropriateness calculation unit (25) for the first ultrasound image and the appropriateness calculated by the appropriateness calculation unit (25) for the second ultrasound image are equal to or greater than a predetermined appropriateness threshold value.

2. The ultrasound diagnostic apparatus according to claim 1,
wherein the first ultrasound image and the second ultrasound image are images in which two of an apical two-chamber cross section, an apical three-chamber cross section, an apical four-chamber cross section, or an apical five-chamber cross section are imaged.

3. The ultrasound diagnostic apparatus according to claim 1 or 2,
wherein the measurement unit (29) measures a left ventricular ejection fraction or a cardiac output as the cardiac function of the subject.

4. The ultrasound diagnostic apparatus according to any one of claims 1 to 3,
wherein the image acquisition unit (32) acquires a third ultrasound image in which a cross section of the heart different from both the cross sections in the first ultrasound image and the second ultrasound image is imaged,
the feature extraction unit (26) extracts a feature of an anatomical structure of the heart from the third ultrasound image,
the consistency calculation unit (27) calculates consistency between the feature of the anatomical structure in the first ultrasound image and the feature of the anatomical structure in the second ultrasound image and the feature of the anatomical structure in the third ultrasound image,
the appropriateness calculation unit (25) calculates appropriateness as a target for measurement for the third ultrasound image, and
the measurement instruction unit (25) starts further measurement of the cardiac function by the measurement unit (29) in a case in which the consistency between the feature of the anatomical structure in the first ultrasound image and the feature of the anatomical structure in the second ultrasound image and the feature of the anatomical structure in the third ultrasound image is equal to or greater than the predetermined consistency threshold value, and the appropriateness calculated by the appropriateness calculation unit (25) for the third ultrasound image is equal to or greater than the predetermined appropriateness threshold value.

5. The ultrasound diagnostic apparatus according to any one of claims 1 to 4,
wherein the feature extraction unit (26) extracts a length of a long axis of a left ventricle as the feature of the anatomical structure of the heart.

6. The ultrasound diagnostic apparatus according to claim 5,
wherein the consistency threshold value is 90%.

7. The ultrasound diagnostic apparatus according to any one of claims 1 to 4,
wherein the feature extraction unit (26) extracts a position of a cardiac apex as the feature of the anatomical structure of the heart.

8. The ultrasound diagnostic apparatus according to claim 7,
wherein the consistency threshold value is 90%.

9. The ultrasound diagnostic apparatus according to any one of claims 1 to 8, further comprising:
an adjustment instruction unit (51) that instructs a user to adjust at least one of a scanning position of the ultrasound probe (1), a posture of the subject, or a breathing method of the subject in a case in which, for a predetermined time, the consistency calculated by the consistency calculation unit (27) between the first ultrasound image and the second ultrasound image is less than the predetermined consistency threshold value or any of the appropriateness calculated by the appropriateness calculation unit (25) for the first ultrasound image or the second ultrasound image is less than the predetermined appropriateness threshold value.

10. A method of controlling an ultrasound diagnostic apparatus, the method comprising:
acquiring first and second ultrasound images in which different cross sections of a heart of a subject are imaged, by transmitting and receiving ultrasound beams using an ultrasound probe (1);
extracting a feature of an anatomical structure of the heart from each of the first ultrasound image and the second ultrasound image;
calculating consistency between the extracted feature of the anatomical structure in the first ultrasound image and the extracted feature of the anatomical structure in the second ultrasound image;
calculating appropriateness as a target for measurement for each of the first ultrasound image and the second ultrasound image; and
executing measurement of a cardiac function of the subject in a case in which the consistency calculated between the first ultrasound image and the second ultrasound image is equal to or greater than a predetermined consistency threshold value, and both the appropriateness calculated for the first ultrasound image and the appropriateness calculated for the second ultrasound image are equal to or greater than a predetermined appropriateness threshold value.
